# EUROPÄISCHE PATENTSCHRIFT

(11) **EP 0 680 768 B1**
(45) Veröffentlichungstag und Bekanntmachung des Hinweises auf die Patenterteilung: **06.10.1999**
(21) Anmeldenummer: 95111049.3
(22) Anmeldetag: 09.08.1991
(51) Int. Cl.: A61M 15/00, A61M 11/00

(54) **Austragvorrichtung für Medien**
Dispensing device for substances
Distributeur de substances

(30) Priorität: 01.09.1990 DE 4027749
(43) Veröffentlichungstag der Anmeldung: 08.11.1995
(62) Teilanmeldung aus: 91113392.4
(73) Patentinhaber: Ing. Erich Pfeiffer GmbH, 78315 Radolfzell (DE)
(72) Erfinder: Wolter, Michael, 8266 Steckborn (CH); Zuckschwerdt, Friedrich, 78315 Radolfzell (DE)
(74) Vertreter: Patentanwälte Ruff, Beier, Schöndorf und Mütschele

(56) Entgegenhaltungen:
- CH-A- 258 145
- GB-A- A24 848

## Beschreibung

Die Erfindung betrifft eine Austragvorrichtung nach dem Oberbegriff des Anspruchs 1. Sie ist zum Austrag von Medien geeignet, die ein einziges Medium, gesondert gespeicherte, gleiche oder ungleiche, fließfähige Medien von ggf. unterschiedlichem Aggregatzustand, sein können, wobei das Medium bevorzugt wenigstens teilweise rieselfähig, z.B. pulver- und/oder puderförmig sein kann.

Zweckmäßig ist die Austragvorrichtung so ausgebildet, daß das Medium mit einem Gasstrom aus einer oder mehreren Medienkammern herausgefördert bzw. herausgerissen wird, wobei die jeweilige Medienkammer unmittelbar durch einen Medienspeicher gebildet sein kann.

Die Austragvorrichtung kann z.B. als Inhalator für pulverförmige Substanzen verwendet werden. Bei ihr kann z.B. eine das Pulver enthaltende Kapsel durch Zerstörung geöffnet und deren Inhalt mit der Atemluft der inhalierenden Person abgesaugt werden.

Die Austragvorrichtung kann auch so ausgebildet sein, daß flüssige oder pulverförmige Stoffe mittels eines Druckgasstromes zur Erzeugung eines Aerosols zerstäubt, verteilt und vermischt werden, jedoch muß hier die Austragvorrichtung an eine externe Druckgasquelle angeschlossen werden, was ihre Benutzbarkeit stark einschränkt.

Ist die Austragvorrichtung mit einer Luftpumpe kombiniert, so kann das Medium mit dem Druckluftstrom nicht aus einer Kammer herausgefördert, sondern der Druckluftstrom lediglich getrennt von dem Medium einer Austragdüse zugeführt und dort erst gemischt werden, so daß das Medium zunächst unabhängig vom Luftstrom über eine gesonderte Pumpe bis zur Austragdüse gefördert wird.

Die GB-A-0 024 848 zeigt eine mit einer Luftpumpe kombinierte Austragvorrichtung, bei welcher in den Medienspeicher am Ende des Medienaustrages Luft gepumpt und so das Medium quer in eine Mischkammer überführt wird, aus welcher es erst beim nächsten Pumphub ausgetragen wird.

Die Austragvorrichtung nach der CH-A-0 258 145 weist am stromabwärts liegenden Ende des Strömungskanals einander nur im selben Längsabschnitt gegenüberliegende Eintrittsöffnungen auf, aus welchen das Medium allein durch Gewichtskraft auf den Vorlageboden einer darunterliegenden Kammer fällt. Wird durch den Strömungskanal Luft gepumpt, so wird sie am Vorlageboden unter Mitnahme des Mediums umgelenkt und trägt das Medium radial gerichtet aus der Austragsöffnung ins Freie aus.

Der Erfindung liegt des weiteren die Aufgabe zugrunde, eine Austragvorrichtung der genannten Art zu schaffen, bei welcher Nachteile bekannter bzw. der beschriebenen Ausbildungen vermieden sind und die insbesondere unabhängig von äußeren Druckgasquellen das wirksame Herausfördern des Mediums aus einer speicherartigen Medienkammer gewährleisten soll.

Erfindungsgemäß sind die Merkmale des Anspruchs 1 vorgesehen. Die Austragvorrichtung kann eine handbetätigbare Vorrichtung zur Erzeugung von Druckgas aufweisen bzw. an eine solche Vorrichtung so anschließbar sein, daß das Druckgas wenigstens teilweise mittelbar und/oder unmittelbar, nämlich während der Betätigung, einer Zone zugeführt werden kann, an welcher es das zunächst nach Art einer Vorlage ruhende Medium aufnimmt, mitreißt und nach außen fördert. Die Austragvorrichtung ist dadurch jederzeit einsetzbar, sie kann sehr kompakt ausgebildet werden, und sie ermöglicht die aerosolartige Ausbringung unterschiedlichster Stoffe verschiedener Aggregatzustände, insbesondere von pharmazeutischen, kosmetischen und ähnlichen Substanzen.

Zweckmäßig weist die Vorrichtung einen gegen eine bzw. entlang einer Vorlage für das Medium gerichteten Injektor bzw. einen Wirbelkanal auf, in dessen Bereich durch Querschnittsverengung verhältnismäßig große Strömungsgeschwindigkeiten und Turbulenzen des Gasstromes erzielt werden, so daß das Medium feinstverteilt und vermischt mitgerissen wird. Eine Druckgasmündung kann dabei etwa entgegen Strömungsrichtung aus diesem Bereich gerichtet sein, und/oder das Medium kann allein durch seine Fließfähigkeit über rasterartig angeordnete Querkanäle dem Bereich der Gasströmung zugeführt und durch Unterdruck in den Querkanälen vom Gasstrom aufgenommen werden.

Vorteilhaft ist eine Dosiereinrichtung für das Medium vorgesehen, so daß je manueller Betätigung nur eine ganz bestimmte Menge an Medium abgegeben und ausgetragen wird. Die Dosiereinrichtung ist zweckmäßig über eine Fließstrecke unmittelbar an einen Medienspeicher angeschlossen und das Medium kann außer durch Gewichtskraft auch dadurch in eine Dosierkammer gefördert werden, daß im entsprechenden Bereich durch den Gasstrom ein geringer Unterdruck erzeugt wird. Die Dosierkammer kann nach Füllung wegabhängig vom Betätigungsweg der Dosiereinrichtung eingangsseitig geschlossen werden, während zwischen der Dosierkammer bzw. der Medienvorlage und der ins Freie bzw. in ein Mundstück führenden Austragöffnung keinerlei Ventil erforderlich ist.

Die Druckgasquelle, wie eine Pumpe, liegt zweckmäßig an dem von der Auslaßöffnung abgekehrten Ende der Medienkammer, an der insbesondere ein Kolben der Druckgasquelle lagestarr unmittelbar befestigt ist. Der Kolben ist vorzugsweise mit einer Schnappverbindung am zugehörigen Ende der Medienkammer befestigt. Dem Strömungskanal ist ein Steuerventil zugeordnet, das insbesondere den Eintrittsöffnungen vorgeschaltet und vorzugsweise dem Gasaustritt zugeordnet ist.

Durch die erfindungsgemäße Ausbildung bedarf es nur einer einzigen Pumpe, nämlich derjenigen für die Druckgaserzeugung, wobei jedoch auch mehrere Pumpen in Serie und/oder parallelgeschaltet vorgesehen sein können. Die Pumpenkammer kann manuell veränderbar sein, und die Pumpe ist insbesondere eine axial unmittelbar an die Medienkammer anschließende Schubkolbenpumpe. Die Pumpenkammer hat vorzugsweise eine Weite, die mindestens so groß wie die Weite der Medienkammer und/oder der übrigen Austragvorrichtung ist. Der Pumpkolben und/oder der Pumpenzylinder ist vorzugsweise an einem Medienspeicher mit mindestens einer Schnappverbindung angeordnet bzw. gelagert. Das Ventil weist einen wenigstens teilweise innerhalb des Strömungskanals liegenden und von der Vorlagefläche ringförmig umgebenen Ventilkörper, wie eine Ventilkugel, auf, der mit einer Ventilfeder belastet ist. Die Ventilfeder liegt vorzugsweise innerhalb des Strömungskanals.

Obwohl andere Betätigungsbewegungen denkbar sind, ist die Austragvorrichtung zweckmäßig ausschließlich durch eine etwa lineare Hubbewegung zu betätigen, wobei ihre voneinander abgekehrten äußeren Endflächen, die durch zwei teleskopartig ineinandergreifende Bauteile gebildet sind, die Handhaben bilden. Einer der Bauteile kann dabei einen wenigstens teilweise freiliegenden und über eine verschließbare Öffnung nachfüllbaren Medienspeicher bilden, welcher zweckmäßig im wesentlichen lagestarr oder axial über eine Steuerstrecke beweglich einen Pumpkolben trägt, der in den anderen, ansonsten die Pumpenkammer begrenzenden Bauteil eingreift. Beide Bauteile sind in einfacher Weise durch Schnappverbindungen aneinander befestigt, so daß sich ein sehr einfacher Aufbau ergibt.

Der Mantel der Medienkammer bildet mit einer einteiligen Stirnwand an einem Ende und mit einem am anderen Ende eingesetzten Deckel einen formstabilen Hohlkörper einer von zwei vormontierten und zur Betätigung der Austragvorrichtung manuell gegeneinander bewegbaren Baugruppen. Die beiden Baugruppen sind insbesondere gegeneinander durch mindestens eine Schnappverbindung axial und/oder gegen Verdrehung gesichert. Vorzugsweise ist in einer einlaßseitig ventilgesteuerten Druckkammer der Druckgasquelle eine vorgespannte Rückstellfeder für die Baugruppen angeordnet.

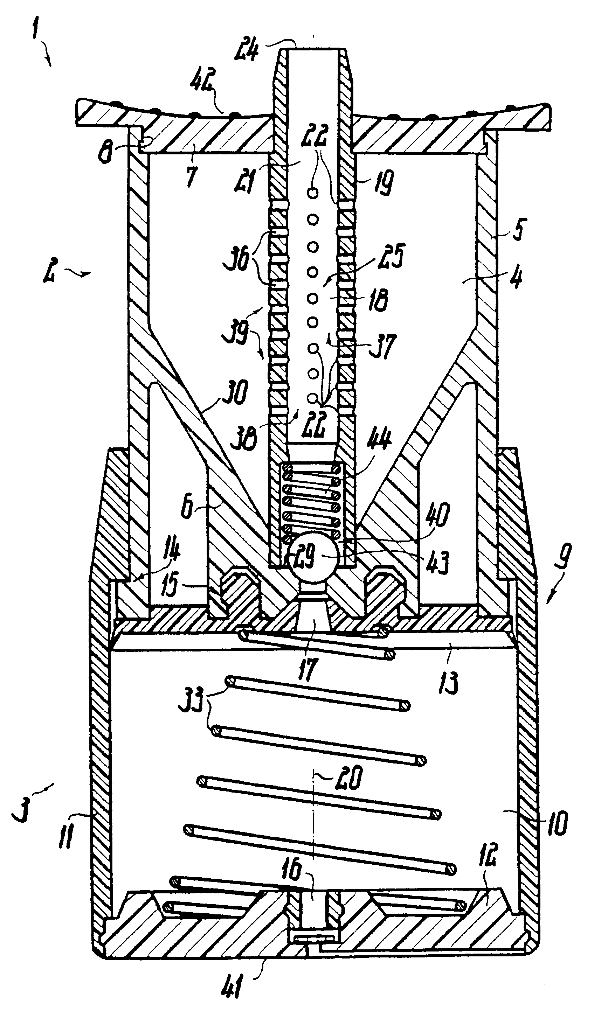

Diese und weitere Merkmale gehen außer aus den Ansprüchen auch aus der Beschreibung und den Zeichnungen hervor, wobei die einzelnen Merkmale jeweils für sich allein oder zu mehreren in Form von Unterkombinationen bei einer Ausführungsform der Erfindung und auf anderen Gebieten verwirklicht sein und vorteilhafte sowie für sich schutzfähige Ausführungen darstellen können, für die hier Schutz beansprucht wird. Ein Ausführungsbeispiel der Erfindung ist in der Zeichnung dargestellt und wird im folgenden näher erläutert. Die Zeichnung zeigt:
eine erfindungsgemäße Austragvorrichtung im Axialschnitt.

Die Austragvorrichtung 1 ist aus nur zwei vormontierten Baugruppen 2, 3 durch eine Steckverbindung zusammengesetzt und weist einen Medienspeicher 4 auf, aus dem je Betätigungshub eine Teilmenge ausgebracht wird. Eine obere Baugruppe 2 bildet mit ihrem äußeren Mantel 5 die äußere Begrenzung des Medienspeichers 4 und des zugehörigen Längsabschnittes der Austragvorrichtung. Der Mantel 5 bildet mit einer einteiligen, ringscheibenförmigen Stirnwand 6 an einem Ende und einem im wesentlichen vollständig versenkt eingesetzten Deckel 7 am anderen Ende einen im wesentlichen formstabilen Hohlkörper. Im eingeschnappten Deckel 7 kann eine exzentrische, mit einem Stopfen zu verschließende Füllöffnung für den Medienspeicher 4 vorgesehen sein.

Die beiden Baugruppen 2, 3 bilden die beiden gegeneinander über einen Pumphub verschiebbar aneinander gelagerten Bestandteile einer Pumpe 9, deren Pumpenkammer 10 von den einander gegenüberliegenden Enden der beiden Baugruppen 2, 3 und einem äußeren Mantel 11 der Baugruppe 3 begrenzt ist, in dessen offenes Ende die Baugruppe 2 eingeschoben ist. Der Medienspeicher 4 und ggf. die Pumpenkammer 10 ist im Querschnitt ringförmig, wobei der lichte Querschnitt und die Außenweite der Pumpenkammer 10 größer als diejenige der Medienkammer 4 sind. Der Mantel 11 kann einteilig mit einer das andere Ende der Austragvorrichtung 1 bildenden Stirnwand 12 ausgebildet sein und nimmt als Kolbenlaufbahn einen dieser gegenüberliegenden, ringförmigen Pumpkolben 13 verschiebbar auf, welcher an der Baugruppe 2 gelagert ist und unmittelbar benachbart zur Stirnwand 6 liegt.

Die beiden Baugruppen 2, 3 sind durch mindestens eine Schnappverbindung 14 gegeneinander axial und gegen Verdrehung gesichert. Jede Schnappverbindung 14 weist am Außenumfang des Mantels 5 einen vorspringenden Schnappnocken auf und kann für dessen Eingriff im Mantel 11 einen Axialschlitz aufweisen, wobei sich an die Axialschlitze die Kolbenlaufbahn bis zur Stirnwand 12 anschließt. Der, ebenfalls über eine Schnappverbindung 15 befestigte, napfförmig mit seinem Rand eine Dichtlippe bildende Pumpkolben 13 kann radial außerhalb seiner Schnappbefestigung im Boden mindestens einen Gaseintritt für die Pumpenkammer 10 aufweisen, der über die Axialschlitze ansaugen kann. Der Gaseintritt 16 ist hier in der Stirnwand 12 vorgesehen und mit einem z.B. federfreien Rückschlagventil versehen. Innerhalb der Schnappverbindung 15 ist im Boden des Pumpkolbens 13 mindestens ein diesen ebenfalls durchsetzender Gasaustritt 17 vorgesehen, über welchen beim Pumphub die Luft aus der Druckkammer 10 in einen ggf. ringförmigen Strömungskanal 18 nach oben gedrückt wird. Der Strömungskanal 18 ist von einem innerhalb des Medienspeichers 4 liegenden und diesen am Innenumfang begrenzenden Kanalmantel 19 begrenzt, der die Stirnwand 6 durchsetzen kann und an dessen zugehörigem Ende der Pumpkolben 13 mit der Schnappverbindung 15 befestigt sein kann. Im wesentlichen alle genannten Bauteile liegen in einer zentralen Achse 20 der Austragvorrichtung 1.

Innerhalb des Strömungskanales 18 kann berührungsfrei ein engerer Auslaßkanal liegen, der von zwei mit den Baugruppen 2, 3 gegeneinander teleskopartig axial verschiebbaren ersten und zweiten Kanalteilen begrenzt sein kann. Ein erster Kanalteil, der den ringförmigen Strömungskanal 18 am Innenumfang begrenzt, kann mit einem Ende radial innerhalb des Gasaustrittes 17 in einer zentralen Öffnung des Pumpkolbens 13 befestigt sein und wie der Kanalmantel 19 vom Pumpkolben bzw. von der Stirnwand 6 frei in den Medienspeicher 4 ragen. Der andere, mit einer Dichtlippe am Innenumfang dieses ersten Kanalteiles geführte zweite Kanalteil kann mit seinem entsprechenden Ende durch eine Schnappverbindung an einer Stirnwand befestigt sein und über einen in dieser liegenden Endabschnitt des Auslaßkanales 21 zu einer Auslaßöffnung 24 führen, die in der Achse 20 oder quer zu dieser gerichtet vorgesehen sein kann.

Zur Zumessung einer mit einem Pumphub auszubringenden Mediencharge ist eine Dosiereinrichtung 25 vorgesehen, die im Bodenbereich des Medienspeichers 4 liegen bzw. eine in Abhängigkeit vom Pumphub zu öffnende und zu schließende Dosierkammer aus zwei ersten und zweiten Kammerteilen aufweisen kann. Der eine erste Kammerteil kann im wesentlichen napfförmig und durch die zugehörige Stirnwand bzw. den Deckel 7 des Medienspeichers 4 gebildet sein. Der andere zweite Kammerteil kann durch den zugehörigen Endabschnitt des Kanalmantels 19 gebildet sein, der bis zum Anschlag an der Bodenfläche 29 eng angepaßt in den Mantel des ersten Kammerteiles einfahrbar wäre und in Ausgangsstellung mit diesem ersten Kammerteil eine ringschlitzförmige Übertrittsöffnung begrenzen würde. An den Mantel bzw. die Bodenfläche des ersten Kammerteils schließt radial nach außen ein ggf. stumpfwinklig konischer Schütt-Trichter 30 an, dessen radial innerer Bereich die eine Begrenzung der Übertrittsöffnung bilden und über welchen das Medium aus dem Medienspeicher 4 auf die Bodenfläche 29 fließen könnte. Die Bodenfläche 29 ist zweckmäßig zur Mitte hin erhöht, so daß das Medium auf ihr in einer ringförmigen Vorlage zu liegen kommt, die etwa deckungsgleich mit dem Strömungskanal 18 ist.

Der zweite Kammerteil kann mit einer Lagerung für den Kanalmantel 19 axial zwischen der beschriebenen Schließstellung für die Übertrittsöffnung und der Ausgangsstellung verschiebbar an der Stirnwand 6 gelagert sein. Ein Stülpmantel kann mit seinem radial äußeren Mantelabschnitt in der Durchtrittsöffnung der Stirnwand 6 und mit seinem radial inneren Mantelabschnitt am Außenumfang des dem Pumpkolben 13 zugehörigen Endes des Kanalmantels 19 befestigt sein, wobei die beiden Mantelabschnitte an ihren vom Pumpkolben 13 abgekehrten und innerhalb des Medienspeichers 4 liegenden Enden über einen Ringabschnitt ineinander übergehen können. Zwischen den beiden Mantelabschnitten kann auf dem Außenumfang des Kanalabschnittes 19 eine Stützfeder angeordnet sein, die sich mit einem Ende am Ringabschnitt des Stülpmantels abstützen und ggf. auch als Rückstellfeder zur Rückstellung des zweiten Kammerteils in die Ausgangsstellung vorgesehen sein kann. Dieser, mit ihrem anderen Ende auf den Pumpkolben 13 axial wirkenden Rückstellfeder könnte eine stärkere Rückstellfeder 33 entgegenwirken, die um den zweiten Kanalteil in der Pumpenkammer 10 vorgespannt zwischen der Stirnwand 12 und dem Pumpkolben 13 angeordnet sein könnte. Die auf den zweiten Kammerteil wirkende Rückstellkraft sollte mindestens so groß sein, daß sie bei Ausgangsstellung der Rückstellfeder 33 die Öffnung der Dosiereinrichtung 25 zuverlässig zuläßt. Je nach Abstimmung der beiden gegeneinander wirkenden Rückstellkräfte könnte die Schließung der Dosiereinrichtung 25 bereits am Anfang des Pumphubes oder erst, z.B. druckabhängig von der Pumpe 9, nach einem ersten Teilweg des Pumphubes erfolgen.

Das untere Ende des Strömungskanals 18 kann eine ringförmig gegen die Bodenfläche 29 gerichtete Mündung bilden, die am Außenumfang vom zweiten Kammerteil, an einer Stirnseite von der vertieften Ringzone der Bodenfläche 29 und am Innenumfang vom zugehörigen Ende des ersten Kanalteils begrenzt sein kann, das bei geschlossener Dosiereinrichtung 25 mit Spaltabstand dem kegelstumpfförmigen zentralen Bereich der Bodenfläche 29 gegenüberliegen würde. Dieses Ende des ersten Kanalteils könnte gleichzeitig einen zentralen Ausgang für das aus der Vorlage- bzw. Dosierkammer durch den Auslaßkanal 21 herauszufördernde Medium bzw. den Eingang des Auslaßkanals 21 bilden. Durch diese Ausbildung kann im Bereich der Ring-Mündung, die in Pumpstellung gegenüber den Querschnitten des Strömungskanals 18 wesentlich verengt wäre, ein Injektor mit einer Umlenkung des Gasstromes an der Bodenfläche 29 um etwa 180° sowie im wesentlichen durch den Mündungsbereich, die geschlossene Dosierkammer und den Ausgangsbereich eine Wirbelkammer zur Aufwirbelung des Mediums gebildet sein.

Die so modifizierte Austragvorrichtung würde zweckmäßig nach folgendem Verfahren arbeiten:

Die voneinander abgekehrten, durch den Deckel 7 und die Stirnwand 12 gebildeten Stirnflächen der Austragvorrichtung 1 dienen als Druck-Handhaben 41, 42 zur einhändigen, manuellen Betätigung der Austragvorrichtung. Die Austragvorrichtung kann dabei verhältnismäßig kleine Abmessungen in der Größenordnung von weniger als 50 mm Durchmesser und weniger als 80 mm Länge haben. Beim manuellen Zusammendrücken der beiden Baugruppen 2, 3 wird zunächst der Zylinder-Mantel 11 entgegen der Kraft der Rückstellfeder 33 gegenüber dem Pumpkolben 13 verschoben, so daß sich in der Pumpenkammer 10 ein Druck aufbaut. Je nachdem, ob dem ringförmig über den Strömungskanal 18 verteilten Gasaustritt 17 nur ein federfreies Rückschlagventil oder ein vorgespanntes Überdruckventil als Steuer-Ventil 40 zugeordnet ist, wird sofort oder erst nach Aufbau eines entsprechenden Überdruckes Druckluft in den Strömungskanal 18 gefördert. Vorher ist durch die ohnehin auftretenden Handhabungsbewegungen über den Trichter 30 aus dem Medienspeicher 14 Medium in die napfförmige Aufnahme des ersten Kammerteils nachgerieselt. Nach Erreichen eines ersten Teilweges des Pumphubes wird der zweite Kammerteil über die Rückstellfeder 33 in die Schließstellung mitgenommen. Die währenddessen geförderte Luft bläst dabei, während die Übertrittsöffnung enger wird, den Sitz für den zweiten Kammerteil 28 von Medium im wesentlichen frei, bis die Übertrittsöffnung geschlossen und der zweite Kammerteil anschlagbegrenzt festgelegt ist.

Die weiterhin geförderte Luft reißt dann das auf der Bodenfläche 29 liegende Medium unter Verwirbelung und Umlenkung mit in den Ausgang, durch den Auslaßkanal 21 und nach außen durch die Auslaßöffnung 24. Gegen Ende des Pumphubes kann die Kraft der Rückstellfeder 33 so stark ansteigen, daß der Boden des ersten Kammerteils unter dem Druck der manuell an ihm angreifenden Betätigungskraft elastisch geringfügig verformt, nämlich gegen den Ausgang bewegt wird, so daß die Mündung ohne zu schließen, kontinuierlich enger und die Strömungsgeschwindigkeit entsprechend höher wird. Dadurch ist eine sehr wirksame vollständige Entleerung der Dosierkammer gewährleistet. Wird danach die Austragvorrichtung freigegeben, so kehrt zunächst die Pumpe 9 unter Schließung des Ventiles 40 zu ihrer Ausgangslage zurück, wonach auch die Dosierkammer wieder öffnet, so daß sofort Medium nachfließen kann. Ggf. bis auf die Federn ist zweckmäßig mindestens einer bzw. sind im wesentlichen alle beschriebenen Bauteile aus Kunststoff hergestellt.

Im Falle des dargestellten Ausführungsbeispiels liegt die Pumpe an dem von der Auslaßöffnung 24 abgekehrten Ende des Medienspeichers 4. Die Pumpenkammer 10 braucht dadurch von keinem Kanal durchsetzt zu sein. Sie weist in der Bodenwand des Pumpkolbens 13 den in Austragrichtung gerichteten Gasaustritt 17 auf, der unmittelbar in das eine Ende des im wesentlichen geradlinigen und den Medienspeicher 4 koaxial durchsetzenden Strömungskanals 18 mündet, dessen anderes Ende die Auslaßöffnung 24 bildet. Das Ventil 40 weist einen z.B. kugelförmigen, wenigstens teilweise innerhalb des zugehörigen Endes des Strömungskanals 18 liegenden Ventilkörper 43 auf, der mit einer innerhalb des Strömungskanals 18 liegenden Ventilfeder 44 zur Schließstellung federbelastet ist.

Der Kanalmantel 19 des Strömungskanals 18 durchsetzt den im Querschnitt im wesentlichen ringförmigen Medienspeicher 4 und ist mit einer Vielzahl über den Umfang sowie in seiner Längsrichtung verteilten, im wesentlichen radialen Querkanälen 36 in Form annähernd geradliniger Manteldurchbrüche versehen, durch welche Medium aus dem Medienspeicher 4 feinverteilt in den Strömungskanal 18 übertreten kann. Ähnlich, wie im Falle der modifizierten Ausbildung durch die Kammerteile ein Schieber-Ventil für das Medium gebildet ist, ist dadurch hier eine Übertrittssteuerung 39 gegeben, wobei die Querschnittsform, Länge, Einzelweite und Gesamtweite der Querkanäle 36 an das Fließverhalten des auszutragenden Mediums angepaßt sind.

Der Kolben 13 ist in diesem Fall lagestarr über eine Schnappverbindung 15 unmittelbar am zugehörigen Ende des Gehäuses des Medienspeichers 4 befestigt. Über eine entsprechende Schnappverbindung 14 sind die beiden Baugruppen 2, 3 miteinander verbunden. Die beiden Schnappverbindungen 14, 15 können an gesonderten, hülsenartig ineinanderliegenden Mantelabschnitten des Speichergehäuses vorgesehen sein, dessen Speicherraum bei 30 zweckmäßig zum Gasaustritt 17 hin trichterartig verjüngt ist. Der Kanalmantel 19 ist zwischen den Stirnwänden des Medienspeichers 4 lagefest eingespannt und durchsetzt den zum Nachfüllen abnehmbaren Deckel 7, der in diesem Fall an dem zur Auslaßöffnung 24 benachbarten Ende des Medienspeichers 4 liegt und die Füllöffnung 8 verschließt. Die Pumpenkammer 10 ist am davon abgekehrten Ende mit einem entsprechenden, eingeschnappten Deckel 12 verschlossen, der den ventilgesteuerten Gaseintritt 16 aufweist.

Bei Betätigung der Austragvorrichtung 1 in der beschriebenen Weise wird zunächst in der Pumpenkammer 10 ein Druck aufgebaut, bis das Ventil 40 öffnet. Eventuell um den Ventilkörper 43 ringförmig abgesunkenes Restmedium wird dadurch schlagartig aufgewirbelt und mitgerissen. Beim Vorbeiströmen des Luftstromes entlang den radial inneren Enden bzw. Eintrittsöffnungen 22 der Querkanäle 36 wird aus diesen Medium bereits vorverteilt herausgerissen, durch Verwirbelung weiter mit dem Luftstrom vermischt und durch die Auslaßöffnung 24 ausgetragen.

## Patentansprüche

1. Austragvorrichtung für fließfähige flüssige bzw. pulverförmige Medien, die mit einem Gasstrom, insbesondere in mehreren aufeinanderfolgenden Chargen mit einer handbetätigbaren Druckgasquelle zur Erzeugung des Gasstromes, aus einer Medienkammer herauszufördern sind, mit Mitteln zur Abförderung des Mediums aus einer das Medium aufnehmenden Vorlage und zur Förderung des Mediums aus einer Auslaßöffnung (24), wobei die Abfördermittel mindestens einen Gasaustritt (17) für den Austritt des das Medium von der Vorlage aufnehmenden und entlang mindestens eines Strömungskanales (18) abfördernden Gasstromes sowie Eintrittsöffnungen (22) für den Eintritt des Mediums in den Strömungskanal (18) aufweisen, dadurch gekennzeichnet, daß die Eintrittsöffnungen (22) in Längsrichtung des Strömungskanales (18) verteilt sind und die Vorlage zur ruhenden Aufnahme des Mediums bilden, wobei eine Übertrittssteuerung (39) mit einem Injektor (37) zur Aufnahme des Mediums aus der Vorlage durch Unterdruck vorgesehen ist.

2. Austragvorrichtung nach Anspruch 1, dadurch gekennzeichnet, daß eine Vielzahl von Eintrittsöffnungen (22) in einer Umfangsrichtung und in der Längsrichtung des Strömungskanales (18) vorgesehen ist, daß insbesondere Eintrittsöffnungen (22) durch Querkanäle (36) gebildet sind und daß vorzugsweise die mit ihren inneren Enden die Eintrittsöffnungen (22) bildenden Querkanäle (36) im wesentlichen radial zum Strömungskanal (18) ausgerichtet und/oder annähernd geradlinig ausgebildet sind.

3. Austragvorrichtung nach Anspruch 1 oder 2, dadurch gekennzeichnet, daß die Eintrittsöffnungen (22) einen Kanalmantel (19) des Strömungskanales (18) durchsetzen, daß insbesondere die Eintrittsöffnungen (22) zur feinverteilten Steuerung des Übertrittes des Mediums in den Strömungskanal (18) nach Querschnittsform, Länge, Einzelweite und Gesamtweite an das Fließverhalten des Mediums angepaßt sind, und daß vorzugsweise der Strömungskanal (18) bis zu der ins Freie führenden Auslaßöffnung (24) eines Auslaßkanales (21) im wesentlichen geradlinig ist.

4. Austragvorrichtung nach Anspruch 3, dadurch gekennzeichnet, daß der Kanalmantel (19) den Strömungskanal (18) von der Medienkammer, wie einem Medienspeicher (4), trennt, daß insbesondere der Kanalmantel (19) innerhalb der Medienkammer liegend und diese an einem Innenumfang begrenzend angeordnet ist, und daß vorzugsweise der Kanalmantel (19) zwischen zwei Stirnwänden (6, 7) der Medienkammer angeordnet ist.

5. Austragvorrichtung nach einem der vorhergehenden Ansprüche, dadurch gekennzeichnet, daß der Strömungskanal (18) in Strömungsrichtung aufsteigend angeordnet ist, daß insbesondere die Medienkammer zu dem in den Strömungskanal (18) mündenden Gasaustritt (17) hin trichterartig verjüngt ist, und daß vorzugsweise eine vom Kanalmantel (19) durchsetzte Stirnwand (7) einer verschließbaren Öffnung zum Nachfüllen der Medienkammer und/oder dem unteren Ende des Strömungskanals (18) eine Vorlagefläche (29) für abgesunkenes Restmedium zugeordnet ist.

6. Austragvorrichtung nach einem der vorhergehenden Ansprüche, dadurch gekennzeichnet, daß der Strömungskanal (18) und die Eintrittsöffnungen (22) mit einer Wirbelkammer (38) verbunden sind, daß insbesondere die Wirbelkammer (38) einen entlang der Eintrittsöffnungen (22) gerichteten Wirbelkanal bildet, und daß vorzugsweise zur Erhöhung der Strömungsgeschwindigkeit und/oder von Turbulenzen im Bereich des Wirbelkanales eine Querschnittsverengung vorgesehen bzw. der Gasaustritt (17) ringförmig über den Strömungskanal (18) verteilt ist.

7. Austragvorrichtung nach einem der vorhergehenden Ansprüche, dadurch gekennzeichnet, daß die Druckgasquelle, wie eine Pumpe (9), an dem von der Auslaßöffnung (24) abgekehrten Ende der Medienkammer liegt, daß insbesondere ein Kolben (13) der Druckgasquelle lagestarr unmittelbar an der Medienkammer befestigt ist, und daß vorzugsweise der Kolben (13) mit einer Schnappverbindung (15) am zugehörigen Ende der Medienkammer befestigt ist.

8. Austragvorrichtung nach einem der vorhergehenden Ansprüche, dadurch gekennzeichnet, daß dem Strömungskanal (18) ein Steuer-Ventil (40) zugeordnet ist, daß insbesondere das Ventil (40) den Eintrittsöffnungen (22) vorgeschaltet ist und daß vorzugsweise das Ventil (40) dem Gasaustritt (17) zugeordnet ist.

9. Austragvorrichtung nach Anspruch 8, dadurch gekennzeichnet, daß das Ventil (40) einen wenigstens teilweise innerhalb des Strömungskanales (18) liegenden und von der Vorlagefläche (29) ringförmig umgebenen Ventilkörper (43), wie eine Ventilkugel, aufweist, daß insbesondere der Ventilkörper (43) mit einer Ventilfeder (44) belastet ist und daß vorzugsweise die Ventilfeder (44) innerhalb des Strömungskanales (18) liegt.

10. Austragvorrichtung nach einem der vorhergehenden Ansprüche, dadurch gekennzeichnet, daß ein Mantel (5) der Medienkammer mit einer einteiligen Stirnwand (6) an einem Ende und mit einem am anderen Ende eingesetzten Deckel (7) einen formstabilen Hohlkörper einer von zwei vormontierten und zur Betätigung der Austragvorrichtung manuell gegeneinander bewegbaren Baugruppen (2, 3) bildet, daß insbesondere die beiden Baugruppen (2, 3) gegeneinander durch mindestens eine Schnappverbindung (14) axial und/oder gegen Verdrehung gesichert sind und daß vorzugsweise in einer einlaßseitig ventilgesteuerten Druckkammer (10) der Druckgasquelle eine vorgespannte Rückstellfeder (33) für die Baugruppen (2, 3) angeordnet ist.

## Claims

1. Discharge apparatus for flowable, fluid or pulverulent media, which are delivered with a gas flow from a medium chamber, particularly in the form of several successive charges, with a manually operable compressed gas source for providing the gas flow, with means for conveying the medium out of a storage means receiving the medium and for feeding the medium out of an outlet opening (24), the conveying away means having at least one gas outlet (17) for the gas flow receiving the medium from the storage means and conveying it away along at least one flow channel (18), as well as inlet openings (22) for the introduction of the medium into the flow channel (18), characterized in that the entrance openings (22) are distributed in the longitudinal direction of the flow channel and form the storage means for the static reception of the medium, a transfer control (39) being provided with an injector (37) for receiving the medium from the storage means by vacuum.

2. Discharge apparatus according to claim 1, characterized in that a plurality of entrance openings (22) is provided in a circumferential direction and in the longitudinal direction of the flow channel (18), that in particular entrance openings (22) are formed by transverse channels (26) and that preferably the transverse channels (36) forming with their inner ends the entrance openings (22) are oriented substantially radially to the flow channel (18) and/or approximately linearly constructed.

3. Discharge apparatus according to claim 1 or 2, characterized in that the entrance openings (22) traverse a channel jacket (19) of the flow channel (18), that in particular the entrance openings (22) for the finely divided control of the transfer of the medium into the flow channel (18) are adapted to the flow behaviour of the medium according to cross-sectional shape, length, single width and total width and that preferably the flow channel (18) is substantially linear up to the outlet opening (24) of an outlet channel (21) leading into the open.

4. Discharge apparatus according to claim 3, characterized in that the channel jacket (19) separates the flow channel (18) from the medium chamber, such as a medium reservoir (4), that in particular the channel jacket (19) is arranged horizontally within the medium chamber and is adjacent thereto at an inner circumference and that preferably the channel jacket (19) is positioned between two end walls (6, 7) of the medium chamber.

5. Discharge apparatus according to one of the preceding claims, characterized in that the flow channel (18) rises in the flow direction, that in particular the medium chamber narrows in funnel-like manner to the gas outlet (17) issuing into the flow channel (18) and that preferably a storage surface (29) for residual medium which has sunk is associated with an end wall (7), traversed by the channel jacket (19), of a closable opening for refilling the medium chamber and/or the lower end of the flow channel (18).

6. Discharge apparatus according to one of the preceding claims, characterized in that the flow channel (18) and entrance openings (22) are connected to a turbulence chamber (38), that in particular the turbulence chamber (38) forms a turbulence channel directed along the entrance openings (22) and that preferably for increasing the flow rate and/or turbulence in the vicinity of the turbulence channel is provided a cross-sectional constriction or the gas outlet (17) is distributed in annular manner over the flow channel (18).

7. Discharge apparatus according to one of the preceding claims, characterized in that the compressed gas source, such as a pump (9), is located at the end of the medium chamber remote from the outlet opening (24), that in particular a piston (13) of the compressed gas source is fixed in positionally rigid manner directly to the medium chamber and that preferably the piston (13) is fixed with a snap connection (15) to the associated end of the medium chamber.

8. Discharge apparatus according to one of the preceding claims, characterized in that with the flow channel (18) is associated a control valve (40), that in particular the valve (40) is positioned upstream of the inlet openings (22) and that preferably the valve (40) is associated with the gas outlet (17).

9. Discharge apparatus according to claim 8, characterized in that the valve (40) has a valve body (43), such as a ball valve at least partly located within the flow channel (18) and surrounded in annular manner by the storage surface (29), that in particular the valve body (43) is loaded with a valve spring (44) and that preferably the valve spring (44) is located within the flow channel (18).

10. Discharge apparatus according to one of the preceding claims, characterized in that a jacket (5) of the medium chamber with a one-piece end wall (6) at one end and with a cover (7) inserted at the other end forms a dimensionally stable hollow body of one of two preassembled subassemblies (2, 3), manually movable against one another for operating the discharge apparatus, that in particular the two subassemblies (2, 3) are secured axially against one another and/or against twisting by at least one snap connection (14) and that preferably a pretensioned restoring spring (33) for the subassemblies (2, 3) is located in an inlet side, valve-controlled pressure chamber (10) of the compressed gas source.

## Revendications

1. Dispositif de distribution pour des matériaux liquides ou encore en poudre aptes à l'écoulement, qui sont à convoyer hors d'une chambre à matériaux avec un jet de gaz, notamment en plusieurs charges consécutives par une source de gaz comprimé, actionnable manuellement pour la production du jet de gaz, avec des moyens pour l'évacuation du matériau hors d'une préchambre, recevant le matériau, et pour le convoiement du matériau hors d'un orifice d'évacuation (24), où les moyen d'évacuation présentent au moins une sortie de gas (17) pour la sortie du jet de gaz, qui ramasse le matériau de la préchambre et l'évacue le long d'au moins un conduit d'écoulement (18), ainsi que des ouvertures d'admission (22) pour l'admission du matériau dans le conduit d'écoulement (18), caractérisé en ce que les ouvertures d'admission (22) sont distribuées en direction longitudinale du conduit d'écoulement (18) et qu'elles forment la préchambre pour le logement au repos du matériau, où est prévue une commande de transfert (39) avec un injecteur (37) pour la réception du matériau de la préchambre par sous-pression.

2. Dispositif de distribution selon la revendication 1, caractérisé en ce que plusieurs ouvertures d'admission (22) sont prévues dans une direction de circonférence et dans la direction longitudinale du conduit d'écoulement (18), en ce que notamment des ouvertures d'admission (22) sont formées par des conduits transversaux (36) et en ce que de préférence les conduits transversaux (36), qui forment avec leurs extrémités intérieures les ouvertures d'admission (22), sont orientés essentiellement de manière radiale par rapport au conduit d'écoulement (18) et/ou sont réalisés approximativement en ligne droite.

3. Dispositif de distribution selon la revendication 1 ou 2, caractérisé en ce que les ouvertures d'admission (22) traversent une enveloppe de conduit (19) du conduit d'écoulement (18), en ce que notamment les ouvertures d'admission (22) sont adaptées pour le contrôle du transfert finement dispersé du matériau dans le conduit d'écoulement (18) d'après la forme de la section transversale, la longueur, l'étendue individuelle et totale à la fluidité du matériau et en ce que de préférence le conduit d'écoulement (18) est en substance rectiligne jusqu'à l'orifice d'évacuation (24) conduisant à l'extérieur, d'un conduit d'évacuation (21).

4. Dispositif de distribution selon la revendication 3, caractérisé en ce que l'enveloppe de conduit (19) sépare le conduit d'écoulement (18) de la chambre à matériaux, tel qu'un réservoir de matériau (4), en ce que notamment l'enveloppe de conduit (19) est disposée de manière à être située à l'intérieur de la chambre à matériaux et de manière à délimiter celle-ci à la circonférence intérieure, et en ce que de préférence l'enveloppe de conduit (19) est disposée entre deux parois frontales (6, 7) de la chambre à matériaux.

5. Dispositif de distribution selon une des revendications précédentes, caractérisé en ce que le conduit d'écoulement (18) est disposé de manière ascendante en direction d'écoulement, en ce que notamment la chambre à matériaux est convergente en forme d'entonnoir en direction de la sortie de gaz (17), qui débouche dans le conduit d'écoulement (18), et en ce que de préférence une paroi frontale (7) traversée par l'enveloppe de conduit (19) est associée à une ouverture à capsuler pour la recharge de la chambre à matériaux et/ou une surface de la préchambre (29) est associée à l'extrémité inférieure du conduit d'écoulement (18) pour le matériau restant tombé vers le bas.

6. Dispositif de distribution selon une des revendications précédentes, caractérisé en ce que le conduit d'écoulement (18) et les ouvertures d'admission (22) sont jointes avec une chambre de turbulence (38), en ce que notamment la chambre de turbulence (38) forme un conduit à turbulence orienté le long des ouvertures d'admission (22), et en ce que de préférence pour l'augmentation de la vitesse d'écoulement et/ou des mouvements tourbillonnaires dans le domaine du conduit à turbulence est prévu un rétrécissement de la section transversale ou encore que la sortie du gaz (17) est distribuée en forme annulaire sur le conduit d'écoulement (18).

7. Dispositif de distribution selon une des revendications précédentes, caractérisé en ce que la source de gaz comprimé, tel qu'une pompe (9), se trouve à l'extrémité opposée de l'orifice d'évacuation (24) de la chambre à matériaux, en ce que notamment un piston (13) de la source de gaz comprimé est fixé en positionnement rigide directement à la chambre à matériaux, et en ce que de préférence le piston (13) est fixé avec un raccordement à enclenchement (15) à l'extrémité correspondante de la chambre à matériaux.

8. Dispositif de distribution selon une des revendications précédentes, caractérisé en ce qu'au conduit d'écoulement (18) est associé une soupape de commande (40), en ce que notamment la soupape (40) est mis en précascade sur les ouvertures d'admission (22) et en ce que de préférence la soupape (40) est associée à la sortie de gaz (17).

9. Dispositif de distribution selon la revendication 8, caractérisé en ce que la soupape (40) présente un corps de soupape (43), tel qu'une sphère d'obturation, le corps se trouvant au moins en partie à l'intérieur du conduit d'écoulement (18) et étant entouré par la surface de préchambre (29) en forme annulaire, en ce que notamment le corps de soupape (43) est chargé par un ressort de soupape (44) et en ce que de préférence le ressort de soupape (44) est situé à l'intérieur du conduit d'écoulement (18).

10. Dispositif de distribution selon une des revendications précédentes, caractérisé en ce qu'une enveloppe (5) de la chambre à matériaux, avec une paroi frontale (6) en une seule pièce à une extrémité et avec un couvercle (7) placé à l'autre extrémité, forme un corps creux indéformable d'un de deux sous-groupes (2,3) prémontés, mobiles manuellement l'un par rapport à l'autre pour l'actionnement du dispositif de distribution, en ce que notamment les deux sous-groupes (2, 3) sont assurés axialement et/ou contre des efforts de torsion l'un par rapport à l'autre par au moins un raccordement à enclenchement (14) et en ce que de préférence dans une chambre sous pression (10), commandée par soupape du côté de l'admission, de la source de gaz comprimé est disposé un ressort de rappel (33) pour les sous-groupes (2, 3).
